# EUROPÄISCHE PATENTSCHRIFT

(11) **EP 2 748 592 B1**
(45) Veröffentlichungstag und Bekanntmachung des Hinweises auf die Patenterteilung: **07.06.2017**
(21) Anmeldenummer: 12766906.7
(22) Anmeldetag: 15.08.2012
(51) Int. Cl.: G01N 23/00, G01V 5/00, G01T 7/00, G01N 23/06

(54) **FLÜSSIGES GEMISCH ZUM TESTEN UND VALIDIEREN VON PRÜFGERÄTEN**
LIQUID MIXTURE USED TO TEST AND VALIDATE TEST DEVICES
MÉLANGE LIQUIDE POUR TESTER ET VALIDER DES APPAREILS DE CONTRÔLE

(30) Priorität: 22.08.2011 DE 102011081328; 10.11.2011 DE 102011118094
(43) Veröffentlichungstag der Anmeldung: 02.07.2014
(73) Patentinhaber: Smiths Heimann GmbH, 65205 Wiesbaden (DE)
(72) Erfinder: SIEDENBURG, Uwe, 55270 Essenheim (DE)
(74) Vertreter: Greif, Thomas
(86) Internationale Anmeldenummer: PCT/EP2012/065951
(87) Internationale Veröffentlichungsnummer: WO 2013/026754

(56) Entgegenhaltungen:
- WO-A1-2009/136677
- D. L. SORBY ET AL: "Dielectric constants of complex pharmaceutical solvent systems. I. Water-ethanol-glycerin and water-ethanol-propylene glycol", JOURNAL OF PHARMACEUTICAL SCIENCES, Bd. 52, Nr. 12, 1. Dezember 1963 (1963-12-01), Seiten 1149-1153, XP55040095, ISSN: 0022-3549, DOI: 10.1002/jps.2600521211
- COX R N ET AL: "A continuous-flow, rapid-mixing, photolabeling technique applied to the acetylcholine receptor", ANALYTICAL BIOCHEMISTRY, ACADEMIC PRESS INC, NEW YORK, Bd. 136, Nr. 2, 1. Februar 1984 (1984-02-01), Seiten 476-486, XP024825300, ISSN: 0003-2697, DOI: 10.1016/0003-2697(84)90247-1 [gefunden am 1984-02-01]
- MICHELLE LE ROUX C ET AL: "Preserving the neurovascular supply in the Hall-Findlay superomedial pedicle breast reduction: an anatomical study", JOURNAL OF PLASTIC, RECONSTRUCTIVE AND AESTHETIC SURGERY, CHURCHILL LIVINGSTONE, GB, Bd. 63, Nr. 4, 1. April 2010 (2010-04-01), Seiten 655-662, XP026932206, ISSN: 1748-6815, DOI: 10.1016/J.BJPS.2009.01.014 [gefunden am 2009-02-25]
- HERZEN J ET AL: "Quantitative phase-contrast tomography of a liquid phantom using a conventional x-ray tube source", OPTICS EXPRESS OPTICAL SOCIETY OF AMERICA USA, Bd. 17, Nr. 12, 2009, Seiten 10010-10018, XP002686810, ISSN: 1094-4087

## Beschreibung

In der deutschen Patentanmeldung DE 10 2011 081 328.4 ist beschrieben, zur Überprüfung von Personen und Objekten, wie Gepäckstücken, auf gefährliche Stoffe, wie Spreng- oder Explosivstoffe, Prüfanlagen zu verwenden, in denen die zu prüfenden Personen oder Objekte in Prüfgeräte von elektromagnetischen Strahlen durchleuchtet oder bestrahlt werden. Bekannterweise werden derartige Prüfanlagen an Flughäfen zur Überprüfung von Passagieren und Gepäckstücken eingesetzt.

Die Prüfanlagen enthalten nach einer bekannten Ausführungsform Prüfgeräte, bei denen die zu überprüfenden Objekte, beispielsweise die Gepäckstücke, von Röntgenstrahlen durchleuchtet oder bestrahlt werden und die transmittierten oder gestreuten Röntgenstrahlen detektiert und ausgewertet werden (DE 10125531-A, DE 19954662-A).

Zur Überprüfung von Personen sind Prüfgeräte bekannt, bei denen die zu überprüfenden Personen mit elektromagnetischen mm-Wellen bestrahlt und die gestreuten mm-Wellen für eine bildhafte Darstellung ausgewertet werden (DE 102005016106-A).

Vor der Inbetriebnahme müssen die Prüfgeräte getestet und validiert werden. Dies erfolgt üblicherweise mit den realen Gefahrstoffen, also den zu detektierenden Sprengstoffen. Die Benutzung von Sprengstoffen ist gesetzlich geregelt, zudem sind sie schwer handhabbar.

Aus dem US-Patent 5,958,299 ist ein Sprengstoff-Simulationsgemisch bekannt, das nicht explosive Bestandteile enthält, wobei die Komponenten so ausgewählt sind, dass die Mischung eine physikalische Form, Dichte, Röntgentransmission und eine effektive Ordnungszahl aufweist, die einem ausgewählten Sprengstoffgemisch entspricht. Mit dem Simulationsgemisch anstelle eines realen Sprengstoffes lässt sich ein Röntgenprüfgerät auf die Detektion des bestimmten Sprengstoffes testen. Als Simulationsgemische sind feste, plastische und gelförmige Zusammensetzungen beschrieben, die sich aus verschiedenen Komponenten aufbauen. Das Dokument WO-A-2009136677 offenbart ein Gemisch, das aus Glycerin, Ethanol und Wasser besteht.

Die wissenschaftliche Veröffentlichung "Quantitative phase-contrast tomography of a liquid phantom using a conventional x-ray tube source", Herzen J et Al, Optics Express Optical Society of America, Bd. 17, Nr. 12, 2009, Seiten 10010-10018, offenbart die Verwendung eines flüssigen Phantoms für Röntgentomographie. An Prüfanlagen wird zunehmend die Anforderung gestellt, auch Flüssigkeitssprengstoffe und sogenannte "home made explosives" zu detektieren. Der Erfindung liegt daher die Aufgabe zugrunde, eine derartige Spreng- oder Explosivstoffe simulierende Materialmischung bereitzustellen, die nicht explosiv, unkritisch in der Handhabung und preisgünstig in der Herstellung ist, sowie sich in einem Prüfgerät zur Überprüfung von Gegenständen oder Personen verhält wie der reale Gefahrstoff.

In der DE 10 2011 081 328.4 wird als Simulationsgemisch ein Gemisch aus Glycerin, Natriumoxid (NaOH) und Wasser verwendet, wobei Glycerin und Natriumhydroxid in einem Gewichtsverhältnis Glycerin / Natriumhydroxid zwischen 6,5 und 3,8 vorliegen.

Nach der Erfindung wird zur Simulation von explosiven Flüssigkeiten ein Gemisch aus Glycerin, Ethanol und Wasser verwendet. Dabei beträgt
vorzugsweise der Anteil von Glycerin in dem Gemisch 33,7 Gew.-% - 48,2 Gew.-%, insbesondere ca. 41,2 Gew.-%. Der Anteil von Ethanol (C₂H₅OH) beträgt vorzugsweise 48,272 Gew.-% - 62,272 Gew.-%, insbesondere ca. 55,272 Gew.-%. Das Gemisch wird durch Wasser auf 100 Gew.-% ergänzt.

Eine bevorzugte Mischung besteht aus 41,2 Gew.-% Glycerin, 55,272 Gew.-% Ethanol (C₂H₅OH) und 3,528 Gew.-% Wasser.

Mit dieser Mischung werden Prüfgeräte getestet und validiert, die dazu bestimmt sind, flüssige Gefahrstoffe zu detektieren. Bei der Validation wird geprüft, ob die Prüfanlage vorbestimmte Anforderungen erfüllt.

Für den Test und die Validation wird in einem zu überprüfenden Gegenstand oder an einer Person das flüssige Simulationsgemisch nach der Erfindung angeordnet. Das Simulationsgemisch besteht aus Glycerin, Ethanol und Wasser.

Die vorstehend beschriebene Grundrezeptur lässt sich unter Beibehaltung der Verhältnisse zwischen Glycerin und Ethanol durch mehr oder weniger Wasser verdünnen oder verdicken, wobei das Gesamtgemisch flüssig bleibt. Durch die unterschiedlichen Anteile von Wasser lassen sich Simulationsgemische zur Simulation von verschiedenen flüssigen Gefahrstoffen herstellen.

In den Prüfgeräten werden die zu überprüfenden Gegenstände, insbesondere Gepäckstücke, mit elektromagnetischen Strahlen durchleuchtet oder bestrahlt. Die transmittierten oder gestreuten Strahlen werden detektiert und ausgewertet.

Eine andere Verwendung eines Gemischs nach der Erfindung erfolgt zum Testen und Validieren von Röntgenprüfgeräten zur Überprüfung von Personen oder Gegenständen, insbesondere von Gepäckstücken, wie sie in der Beschreibungseinleitung als bekannt beschrieben werden.

Ebenso kann das Simulationsgemisch nach der Erfindung verwendet werden, um Prüfgeräte zu testen und validieren, in denen elektromagnetische mm-Wellen zur Überprüfung von Personen oder Gegenständen verwendet werden.

## Patentansprüche

1. Verwendung eines flüssigen Gemischs zum Testen und Validieren von Röntgenprüfgeräten zur Überprüfung von Gegenständen oder Personen, das Glycerin enthält,
**dadurch gekennzeichnet, dass** es aus einem Gemisch aus Glycerin, Ethanol und Wasser besteht.

2. Verwendung eines flüssigen Gemischs zum Testen und Validieren von Prüfgeräten, in denen elektromagnetische mm-Wellen verwendet werden, zur Überprüfung von Gegenständen oder Personen, das Glycerin enthält,
**dadurch gekennzeichnet, dass** es aus einem Gemisch aus Glycerin, Ethanol und Wasser besteht.

3. Verwendung eines flüssigen Gemischs nach Anspruch 1 oder 2, **dadurch gekennzeichnet, dass** der Anteil von Glycerin 33,7 Gew.-% - 48,2 Gew.-%, insbesondere ca. 41,2 Gew.-%, der Anteil von Ethanol 48,272 Gew.-% - 62,272 Gew.-%, insbesondere ca. 55,272 Gew.-%, beträgt und der restliche Anteil zu 100 Gew.-% aus Wasser besteht.

## Claims

1. Use of a liquid mixture for testing and validating x-ray screening devices for inspecting objects or persons which contains glycerol,
**characterized in that** it consists of a mixture of glycerol, ethanol and water.

2. Use of a liquid mixture for testing and validating screening devices using electromagnetic mm waves for inspecting objects or persons which contains glycerol,
**characterized in that** it consists of a mixture of glycerol, ethanol and water.

3. Use of a liquid mixture according to Claim 1 or 2, **characterized in that** the proportion of glycerol is 33.7 wt% - 48.2 wt%, especially *circa* 41.2 wt%, the proportion of ethanol is 48.272 wt% - 62.272 wt%, especially *circa* 55.272 wt%, and the remaining proportion to 100 wt% consists of water.

## Revendications

1. Utilisation d'un mélange liquide pour tester et valider des appareils de test par rayons X pour le contrôle d'objets ou de personnes, qui contient du glycérol, **caractérisé en ce qu'**il est constitué par un mélange de glycérol, d'éthanol et d'eau.

2. Utilisation d'un mélange liquide pour tester et valider des appareils de test, dans lesquels on utilise des ondes mm électromagnétiques, pour le contrôle d'objets ou de personnes, qui contient du glycérol, **caractérisé en ce qu'**il est constitué par un mélange de glycérol, d'éthanol et d'eau.

3. Utilisation d'un mélange liquide selon la revendication 1 ou 2, **caractérisé en ce que** la proportion de glycérol est de 33,7% en poids à 48,2 % en poids, en particulier d'environ 41,2% en poids, la proportion d'éthanol est de 48,272% en poids à 62,272% en poids, en particulier d'environ 55,272 % en poids, et la proportion résiduelle jusqu'à 100% en poids est constituée par de l'eau.
